# FASCICULE DE BREVET EUROPEEN

(11) **EP 2 367 842 B1**
(45) Date de publication et mention de la délivrance du brevet: **18.03.2015**
(21) Numéro de dépôt: 09804287.2
(22) Date de dépôt: 23.12.2009
(51) Int. Cl.: C07K 5/10, C07K 7/06, A61K 38/07, A61K 38/08, A61K 8/64, A61Q 17/00, A61Q 19/00

(54) **PEPTIDE DERIVE D1HMG-COA REDUCTASE ET COMPOSITION COSMETIQUE OU PHARMACEUTIQUE LE CONTENANT**
VON HMG-COA-REDUKTASE ABGELEITETES PEPTID UND DIESES ENTHALTENDE KOSMETISCHE ODER PHARMAZEUTISCHE ZUSAMMENSETZUNG
HMG-COA REDUCTASE DERIVED PEPTIDE AND COSMETIC OR PHARMACEUTICAL COMPOSITION CONTAINING SAME

(30) Priorité: 23.12.2008 FR 0807362
(43) Date de publication de la demande: 28.09.2011
(73) Titulaire: ISP Investments Inc., Wilmington, DE 19805 (US)
(72) Inventeur: DAL FARRA, Claude, Kerhonkson NY 12446 (US); DOMLOGE, Nouha, 06560 Valbonne (FR); BOTTO, Jean-Marie, 06560 Valbonne (FR)
(74) Mandataire: Macquet, Christophe
(86) Numéro de dépôt international: PCT/FR2009/001475
(87) Numéro de publication internationale: WO 2010/072926

(56) Documents cités:
- EP-A2- 1 152 062
- WO-A1-03/023067
- WO-A2-03/087831
- WO-A2-2004/031211
- WO-A2-2005/047328
- WO-A2-2005/080985
- FR-A1- 2 868 309
- GOURLEY D G ET AL: "HMG-CoA reductase: A novel target for antimicrobial chemotherapy." ABSTRACTS OF THE INTERSCIENCE CONFERENCE ON ANTIMICROBIAL AGENTS AND CHEMOTHERAPY, vol. 43, 2003, page 219, XP9035587 & 43RD ANNUAL INTERSCIENCE CONFERENCE ON ANTIMICROBIAL AGENTS AND CHEMOTHERAPY; CHICAGO, IL, USA; SEPTEMBER 14-17, 2003

### SEQUENCE LISTING

<110> ISP Investments INC.
<120> PEPTIDE DERIVE D'HMG-CoA REDUCTASE ET COMPOSITION COSMETIQUE OU PHARMACEUTIQUE LE CONTENANT
<130> BV PCT 08-110
<150> 08 07362 <151> 2008-12-23
<160> 7
<170> PatentIn version 3.3
<210> 1
   <211> 10
   <212> PRT
   <213> Artificial
<220>
   <223> Synthetic peptide
<400> 1
   Met Ala Gly Glu Leu Ser Leu Met Ala Ala
   1 5 10
<210> 2
   <211> 9
   <212> PRT
   <213> Artificial
<220>
   <223> Synthetic peptide
<400> 2
   Gly Val Gly Glu Leu Ser Ile Ser Ala
   1 5
<210> 3
   <211> 8
   <212> PRT
   <213> Artificial
<220>
   <223> Synthetic peptide
<400> 3
   Ile Gly Glu Leu Ser Leu Ala Ala
   1 5
<210> 4
   <211> 5
   <212> PRT
   <213> Artificial
<220>
   <223> Synthetic peptide
<400> 4
   Ala Gly Glu Leu Ser
   1 5
<210> 5
   <211> 5
   <212> PRT
   <213> Artificiel
<220>
   <223> Synthetic peptide
<220>
   <221> MOD_RES
   <222> (5)..(5)
   <223> AMIDATION
<400> 5
   Ala Gly Glu Leu Ser
   1 5
<210> 6
   <211> 5
   <212> PRT
   <213> Artificial
<220>
   <223> Synthetic peptide
<400> 6
   Ile Gly Glu Leu Ser
   1 5
<210> 7
   <211> 5
   <212> PRT
   <213> Artificiel
<220>
   <223> Synthetic peptide
<220>
   <221> MOD_RES
   <222> (5)..(5)
   <223> AMIDATION
<400> 7
   Ile Gly Glu Leu Ser
   1 5

## Description

La présente invention se situe dans le domaine cosmétique et pharmaceutique, et plus particulièrement dans le domaine de la dermatologie. La présente invention concerne des peptides dérivés de l'enzyme 3-hydroxy-3methyl-glutaryl Co-A réductase humaine (HMG-CoA réductase humaine) et plus particulièrement des peptides de formule générale (I) :

R₁-(AA)ₙ-X₁-Gly-Glu-Leu-Ser-X₂-X₃-(AA)ₚ-R₂.

La présente invention concerne également une composition cosmétique ou pharmaceutique, comprenant un peptide de formule générale (I), utilisé seul ou en association avec au moins un autre principe actif, dans un milieu dans un milieu physiologiquement adapté. L'invention est également relative à l'utilisation de ce nouveau peptide, en tant que principe actif activateur de la HMG-CoA réductase humaine, dans une composition cosmétique destinée à renforcer la fonction barrière cutanée et à stimuler la différenciation épidermique.

La fonction première de l'épiderme est de constituer une barrière entre l'environnement extérieur et le milieu intérieur. C'est la couche la plus externe de l'épiderme, *le stratum corneum,* qui assure cette fonction. Il est composé de kératinocytes au stade ultime de leur différenciation, les cornéocytes, scellés les uns aux autre par un épais ciment intercellulaire, à la fois souple et imperméable. On distingue ainsi dans le *stratum corneum* un compartiment cellulaire, constitué des cornéocytes et un compartiment extracellulaire principalement constitué de lipides, organisés en structures multilamellaires.

Le contenu lipidique du *stratum corneum* humain est estimé à 15% d'ester de cholestérol, 16 % d'acides gras libres saturés à longues chaînes, 32 % de cholestérol, 37 % de céramides, bien que les variations inter-individuelles soient assez importantes (Norlen L. et al. J. Invest. Dermatol. 1999; 112(1) p. 72-77). Ces lipides sont synthétisés par les kératinocytes des couches intermédiaires de l'épiderme, et sécrétés dans des organites spécialisés appelés « corps lamellaires » ou corps de Odland. En particulier, l'épiderme est un site très actif pour la synthèse du cholestérol. L'étape limitante de cette synthèse, et la plus finement régulée, est la conversion du 3-hydroxy-3-methylglutaryl- Coenzyme A (HMG-CoA) en mévalonate. Cette étape est catalysée par une enzyme membranaire dénommée HMG-CoA-réductase (E.C. 1.1.1.34). Les données de séquençage du génome humain montrent qu'il existe au moins 2 isoformes de HMG-CoA réductase, codées par un gène unique, localisé sur le chromosome 5 (Luskey et al., J Biol Chem., 1985 260(18), p.10271-7).

Dans la peau, le cholestérol joue un rôle dans la fluidité des membranes et en particulier, il semble assurer la mobilité des chaînes hydrocarbonées dans les bicouches lipidiques (Martini M.C., Pathol. Biol. 2003, (51), p. 267-270). Ainsi, en situation physiologique, le cholestérol est synthétisé à un niveau nécessaire pour maintenir une homéostasie. En revanche, à la suite d'une altération brutale de la barrière cutanée, on observe une augmentation importante et rapide de la synthèse de cholestérol, associée à une augmentation de l'expression et de l'activité de la HMG-CoA réductase (Menon G.K. et al., J. Lipid, Res., 1985, (26), P. 418-427).

Le rôle clé de l'HMG-CoA réductase en a fait une cible de choix pour moduler l'expression du cholestérol dans l'organisme. On a ainsi développé une classe de composés pharmacologiques destinés à inhiber l'HMG-CoA réductase dans le but d'abaisser le cholestérol circulant, dénommés statines. Cet effet inhibiteur des statines se manifeste également dans la peau humaine. En effet, l'administration expérimentale de statines par voie topique perturbe la fonction barrière de la peau (Proksch E. et al., British J. Dermatol., 1993, (128), p. 473-482). Ces résultats confirment l'importance du cholestérol dans la fonction barrière épidermique et le rôle central de l'HMG-CoA réductase dans la modulation de sa synthèse.

Au cours du vieillissement cutané, l'intégrité de la barrière cutanée ainsi que ses capacités de réparation s'altèrent. On observe une déficience globale en lipides, aboutissant à une diminution des multicouches lipidiques du compartiment extracellulaire du *stratum corneum.* Ces changements fonctionnels sont en corrélation avec une susceptibilité accrue des peaux âgées aux agressions extérieures (Ghadially R. et al., J Clin Invest., 1995 (95 (5), p. 2281-90).

Indépendamment du vieillissement intrinsèque ou photo-induit, des altérations de la barrière cutanée peuvent se produire lors d'agressions extérieures.

On entend par l'expression « agression extérieure », les agressions que peut produire l'environnement. A titre d'exemple, on peut citer des agressions telles que la pollution, les UV, ou encore les produits à caractère irritant tels que les tensioactifs, les conservateurs ou les parfums, les agressions mécaniques, telles que les abrasions, le rasage ou l'épilation. Par pollution, on entend aussi bien la pollution « extérieure » due par exemple aux particules de diesel, à l'ozone ou aux métaux lourds, que la pollution « intérieure » qui peut être due notamment aux émissions de solvants de peintures, de colles, ou de papier-peints (tels que toluène, styrène, xylène ou benzaldéhyde), ou bien encore la fumée de cigarette. La sécheresse de l'atmosphère est également une cause importante d'agression cutanée. Ces agressions extérieures aboutissent à une altération de la fonction barrière qui se traduit par un inconfort cutané, des phénomènes sensoriels désagréables, tels que des tiraillements ou des démangeaisons, voire des fragilités excessives et des rougeurs.

Dans ce contexte, il est souhaitable de chercher à prévenir l'altération ou à rétablir la fonction barrière de l'épiderme. Dans ce domaine particulier, l'apport direct de substituts lipidiques, tels que les céramides (EP 1272148, US2007576937) ou certains dérivés du cholestérol (FR 2 789 312) a largement été décrit. D'autre part, l'utilisation d'huiles végétales pour activer la synthèse des lipides cutanés a également été décrite (EP 1 707 189). Dans le domaine de la cosmétique, le ciblage moléculaire de l'HMG-CoA réductase a déjà été exploité mais dans le but d'inhiber cette enzyme-clé, par exemple en utilisant des statines, déjà connues pour leurs propriétés inhibitrices de HMGCOA, dans le but d'obtenir un effet antivieillissement, (EP 0738510). Toutefois, à ce jour, aucun document ne décrit ou ne suggère qu'un peptide dérivé de la HMG-CoA réductase humaine peut avoir des propriétés intéressantes pour renforcer la fonction barrière cutanée et pour stimuler la différenciation épidermique. Il a maintenant été envisagé d'activer l'HMG-CoA réductase dans le but de renforcer la fonction barrière et la différenciation de l'épiderme. Il est également possible d'améliorer par cette action certains dysfonctionnements pathologiques liés à la fonction barrière (peaux hypersensibles, irritées, ou réactives, eczéma atopique).

La présente invention a pour principal objectif de fournir un nouveau principe actif, capable de renforcer la fonction barrière cutanée, de stimuler la différenciation épidermique et de prévenir ainsi les manifestations du vieillissement cutané ou de protéger la peau des agressions extérieures. Les inventeurs ont en effet mis en évidence une activité cosmétique et thérapeutique, notamment dermatologique, de peptides dérivés de la HMG-CoA réductase humaine.

Il a en particulier été mis en évidence que ces peptides, lorsqu'ils sont appliqués sur la peau, renforcent la fonction barrière de l'épiderme et stimulent la différenciation épidermique. Ces propriétés ont été démontrées par une meilleure protection du tissu cutané vis-à-vis des agressions extérieures et une augmentation de la production de lipides constitutifs de la couche cornée.

On entend par « principe actif activateur de la HMG-CoA réductase ou capable d'activer la HMG-CoA réductase humaine», tout peptide ou dérivé biologiquement actif capable d'augmenter l'activité de la HMG-CoA réductase, soit par l'augmentation de la synthèse protéique de la HMG-CoA réductase (par modulation directe ou indirecte de l'expression génique de la HMG-CoA réductase), soit par l'augmentation de l'activité enzymatique de la HMG-CoA réductase, soit par d'autres processus biologiques tels que la stabilisation de la protéine HMG-CoA réductase ou encore la stabilisation des transcrits d'ARN messager.

On entend par peau, l'ensemble des tissus de recouvrement constituant la peau et les muqueuses.

On entend par « application topique », le fait d'appliquer ou d'étaler le principe actif selon l'invention, ou une composition le contenant, à la surface de la peau.

On entend par « physiologiquement acceptable » que le principe actif selon l'invention, ou une composition le contenant, est approprié pour entrer en contact avec la peau sans provoquer de réactions de toxicité ou d'intolérance.

Ainsi, l'invention a pour objet premier un peptide dérivé de la HMG-CoA réductase humaine, de formule générale (I)

R₁-(AA)n-X₁-Gly-Glu-Leu-Ser-X₂-X₃-(AA)ₚ-R₂

dans laquelle,
X₁ est l'alanine ou la valine ou l'isoleucine,
X₂ est la leucine ou l'isoleucine ou aucun acide aminé,
X₃ est la méthionine ou la serine ou l'alanine ou aucun acide aminé,
AA représente un acide aminé quelconque, ou un de ses dérivés, et n et p sont des nombres entiers compris entre 0 et 4,
R₁ représente la fonction aminé primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄., caractérisé en ce qu'il correspond à une des séquences suivantes:
   (SEQ ID n°1) Met-Ala-Gly-Glu-Leu-Ser-Leu-Met-Ala-Ala
   (SEQ ID n°2) Gly-Val-Gly-Glu-Leu-Ser-Ile-Ser-Ala
   (SEQ ID n°3) Ile-Gly-Glu-Leu-Ser-Leu-Ala-Ala
   (SEQ ID n°4) Ala-Gly-Glu-Leu-Ser
   (SEQ ID n°5) Ala-Gly-Glu-Leu-Ser-NH2
   (SEQ ID n°6) Ile-Gly-Glu-Leu-Ser
   (SEQ ID n°7) Ile-Gly-Glu-Leu-Ser-NH2.

L'expression « peptide dérivé de la HMG-CoA réductase humaine» désigne tout fragment peptidique biologiquement actif dont la séquence en acides aminés est, entièrement ou partiellement analogue ou homologue de la séquence peptidique de la HMG-CoA réductase humaine.

Par l'expression « biologiquement actif », on entend « qui possède une activité *in vivo* ou *in vitro* caractéristique de l'activité du principe actif selon l'invention ».

Selon un mode de réalisation particulièrement intéressant, le peptide biologiquement actif correspond à la séquence SEQ ID n°4.

Selon un autre mode de réalisation particulièrement intéressant, le peptide biologiquement actif correspond à la séquence SEQ ID n°5.

Dans l'invention, le terme « acide aminé » se réfère ici à tout acide organique naturel ou non naturel ayant la formule :

-NHR-CR-C(O)-O-

où chaque -R est indépendamment sélectionné entre un hydrogène et un groupement alkyl ayant entre 1 et 12 atomes de carbone. Préférentiellement, au moins un groupement -R de chaque acide aminé est un hydrogène. Par le terme « alkyl », on entend ici une chaîne carbonée pouvant être linéaire ou ramifiée, substituée (mono- ou poly-) ou non-substituée ; saturée, mono-saturée (une double ou triple liaison dans la chaîne) ou poly-insaturée (deux ou plusieurs doubles liaisons, deux ou plusieurs triples liaisons, une ou plusieurs doubles liaisons et une ou plusieurs triples liaisons dans la chaîne).

Le terme « peptide » désigne un enchaînement de deux ou plusieurs acides aminés liés entre eux par des liaisons peptidiques ou par des liaisons peptidiques modifiées.

Par « peptide », il faut entendre le peptide naturel ou synthétique de l'invention tel que décrit ci-dessus, ou au moins l'un de ses fragments, qu'il soit obtenu par protéolyse ou de manière synthétique, ou encore tout peptide naturel ou synthétique dont la séquence est totalement ou partiellement constituée par la séquence du peptide précédemment décrit.

De façon à améliorer la résistance à la dégradation, il peut être nécessaire d'utiliser une forme protégée du peptide selon l'invention. La forme de protection doit évidemment être une forme biologiquement compatible et doit être compatible avec une utilisation dans le domaine des cosmétiques ou de la pharmacie.

De nombreuses formes de protection biologiquement compatibles peuvent être envisagées. Elles sont bien connues de l'homme du métier comme, par exemple, l'acylation ou l'acétylation de l'extrémité amino-terminale, ou l'amidation ou l'estérification de l'extrémité carboxy-terminale. Ainsi, l'invention concerne une composition telle que définie précédemment, caractérisée par le fait que le peptide de séquence SEQ ID n°1 à SEQ ID n°7 est sous forme protégée ou non. On peut utiliser une protection basée sur une substitution sur l'extrémité amino-terminale par un groupement acétyle, un groupement benzoyle, un groupement tosyle ou un groupement benzyloxycarbonyle. De préférence, on utilise une protection basée sur l'amidation de la fonction hydroxyle de l'extrémité carboxy-terminale par un groupement NYY avec Y représentant une chaîne alkyle de C₁ à C₄, ou l'estérification par un groupement alkyle. Il est également possible de protéger les deux extrémités du peptide.

Les dérivés de peptides concernent aussi les acides aminés et les peptides reliés entre eux par une liaison pseudo-peptidique. On entend par « liaison pseudo-peptidique », tous les types de liaisons susceptibles de remplacer les liaisons peptidiques « classiques ».

Dans le domaine des acides aminés, les molécules ont une géométrie telle, qu'elles peuvent théoriquement se présenter sous la forme d'isomères optiques différents. Il existe ainsi une conformation moléculaire de l'acide aminé (AA) qui dévie à droite le plan de polarisation de la lumière (conformation dextrogyre ou D-aa), et une conformation moléculaire de l'acide aminé (aa) qui dévie à gauche le plan de polarisation de la lumière (conformation lévogyre ou L-aa). Les acides aminés naturels sont toujours de conformation lévogyre, en conséquence, un peptide d'origine naturelle ne sera constitué que d'acides aminés de type L-aa. Cependant, la synthèse chimique en laboratoire permet de préparer des acides aminés ayant les deux conformations possibles. A partir de ce matériel de base, il est possible d'incorporer lors de la synthèse de peptide aussi bien des acides aminés sous forme d'isomères optiques dextrogyre que lévogyre. Ainsi, les acides aminés constituant le peptide selon l'invention peuvent être sous configuration L- et D- ; de manière préférentielle, les acides aminés sont sous forme L. Le peptide selon l'invention peut donc être sous forme L-, D- ou DL-.

Le peptide de formule générale (I) selon l'invention de séquence SEQ ID n°1-7 peut être obtenu soit par synthèse chimique classique (en phase solide ou en phase homogène liquide), soit par synthèse enzymatique (Kullman et al., J. Biol. Chem. 1980, 225, 8234), à partir d'acides aminés constitutifs ou de leurs dérivés.

Le peptide selon l'invention peut être d'origine naturelle ou synthétique. Préférentiellement selon l'invention, le peptide est obtenu par synthèse chimique.

Selon l'invention, le principe actif peut être un peptide unique ou un mélange de peptides.

Selon l'invention, ledit peptide ou mélange de peptides peut être utilisé à titre de médicament.

Selon un mode de réalisation avantageux de l'invention, le principe actif selon l'invention est préalablement solubilisé dans un ou plusieurs solvants physiologiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon encore un autre mode de réalisation avantageux de l'invention, le principe actif selon l'invention est préalablement solubilisé dans un vecteur cosmétique ou pharmaceutique comme les liposomes, ou adsorbé sur des polymères organiques poudreux, des supports minéraux comme les talcs et bentonites, et plus généralement solubilisé dans, ou fixé sur, tout vecteur physiologiquement acceptable.

L'invention a pour second objet une composition cosmétique, pharmaceutique, et notamment dermatologique, comprenant, dans un milieu physiologiquement adapté, un peptide de formule générale (I) de séquence SEQ ID n°1-7, en tant que principe actif, seul ou en association avec au moins un autre principe actif.

Il est bien évident que l'invention s'adresse aux mammifères en général, et plus particulièrement aux êtres humains.

Selon un mode de réalisation avantageux de l'invention, le principe actif selon l'invention est présent dans les compositions de l'invention à une concentration comprise entre 0,0005 et 500 ppm (parties par million) environ, et préférentiellement à une concentration comprise entre 0,01 et 5 ppm environ par rapport au poids total de la composition finale.

La composition utilisable selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non d'un rinçage, ou encore sous forme de shampooing. Ainsi, le principe actif selon l'invention pourra avantageusement être utilisé dans les soins antipelliculaires du cuir chevelu.

Elle peut également se présenter sous forme de teinture ou de mascara à appliquer au pinceau ou au peigne, en particulier sur les cils, les sourcils ou les cheveux.

Il est bien entendu que le principe actif selon l'invention peut être utilisé seul ou bien en association avec au moins un autre principe actif, dans une composition cosmétique ou pour la préparation d'une composition pharmaceutique et/ou dermatologique. Avantageusement, les compositions utilisables selon l'invention contiennent en outre divers principes actifs protecteurs ou antivieillissement, destinés, notamment, à la prévention et/ou au traitement des désordres liés au vieillissement.

Les compositions selon l'invention pourront être appliquées par toute voie appropriée, notamment orale, parentérale ou topique externe, et leur formulation sera adaptée par l'homme du métier, en particulier pour des compositions cosmétiques ou dermatologiques. Avantageusement, les compositions selon l'invention sont destinées à une administration par voie topique cutanée. Ces compositions doivent donc contenir un milieu physiologiquement acceptable, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, antioxydants, colorants, conservateurs, parfums.

Selon une autre forme de l'invention, les compositions seront appropriées à une administration orale pour un usage pharmaceutique. Ainsi, les compositions pourront notamment se présenter sous forme de comprimés, capsules, gélules, pâtes à mâcher, poudres à consommer telles quelles ou à mélanger extemporanément avec un liquide, sirops, gels, et toute autre forme connue de l'homme du métier. Elles contiendront des excipients de formulation appropriés, tels que colorants, édulcorants, aromatisants, agents de charge, liants, conservateurs.

Ces compositions pourront notamment se présenter sous forme d'une solution aqueuse, hydroalcoolique ou huileuse ; d'une émulsion huile-dans-eau, eau-dans-huile ou émulsions multiples; elles peuvent aussi se présenter sous forme de crèmes, de suspensions, ou encore de poudres, adaptées à une application sur la peau, les muqueuses, les lèvres et/ou les phanères. Ces compositions peuvent être plus ou moins fluides et avoir l'aspect d'une crème, d'une lotion, d'un lait, d'un sérum, d'une pommade, d'un gel, d'une pâte ou d'une mousse. Elles peuvent aussi se présenter sous forme solide, comme un stick, ou être appliquées sur la peau sous forme d'aérosol. Elles peuvent être utilisées comme produit de soin et/ou comme produit de maquillage de la peau.

Ces compositions comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de manière à ne pas nuire aux propriétés avantageuses recherchées de la composition selon l'invention. Ces adjuvants peuvent, par exemple, correspondre à 0,01 à 20 % du poids total de la composition. Lorsque la composition de l'invention est une émulsion, la phase grasse peut représenter de 5 à 80 % en poids et de préférence de 5 à 50 % en poids par rapport au poids total de la composition. Les émulsionnants et co-émulsionnants utilisés dans la composition seront choisis parmi ceux classiquement utilisés dans le domaine considéré. Par exemple, ils peuvent être utilisés en une proportion allant de 0,3 à 30 % en poids, par rapport au poids total de la composition.

L'invention a pour troisième objet l'utilisation, dans une composition cosmétique, d'une quantité efficace de peptide de formule générale (I) de séquence SEQ ID n°1-7, en tant que principe actif activateur de la HMG-CoA réductase humaine.

La quantité efficace de principe actif correspond à la quantité nécessaire pour obtenir le résultat recherché, à savoir, activer la HMG-CoA réductase, dans le but d'améliorer la fonction barrière de l'épiderme et de stimuler la différenciation épidermique.

On entend par « renforcer la fonction barrière cutanée et stimuler la différenciation épidermique », l'amélioration de la capacité de protection de la couche cornée et l'augmentation de l'expression de marqueurs biologiques de différenciations, tels que les kératines.

Ainsi, ledit principe actif, grâce à ses propriétés particulières, pourra être utilisé dans une composition cosmétique destinée à renforcer la fonction barrière cutanée et à stimuler la différenciation épidermique.

D'autre part, le peptide de formule générale (I) de séquence SEQ ID n°1-7 pourra être utilisé avantageusement, en tant que principe actif, dans une composition cosmétique destinée à lutter de manière préventive et/ou curative contre les signes du vieillissement cutané, et plus particulièrement le vieillissement photo-induit (photo-vieillissement). Par manifestations cutanées du vieillissement, on entend toutes modifications de l'aspect extérieur de la peau et des phanères dues au vieillissement comme, par exemple, les rugosités superficielles de la couche cornée, les rides et ridules, mais également toute modification interne de la peau qui ne se traduit pas systématiquement par un aspect extérieur modifié comme, par exemple, l'amincissement du derme ou toute autre dégradation interne de la peau consécutive à une exposition aux rayonnements ultraviolets (UV).

Selon un autre aspect de l'invention, le peptide de formule générale (I) de séquence SEQ ID n°1-7 pourra être utilisé avantageusement, en tant que principe actif, dans une composition cosmétique destinée à protéger la peau contre tous types d'agressions extérieures.

En particulier, l'invention a pour objet l'utilisation d'une composition cosmétique comprenant une quantité efficace de peptide selon l'invention pour prévenir ou traiter les dommages causés à la peau par des traitements mécaniques tels que le rasage ou l'épilation.

En particulier, l'invention a pour objet l'utilisation d'une composition cosmétique comprenant une quantité efficace de peptide selon l'invention, pour prévenir ou traiter les dommages causés à la peau par des conditions climatiques extrêmes ou des variations brutales de températures et d'hygrométrie.

D'autres avantages et caractéristiques de l'invention apparaîtront mieux à la lecture des exemples donnés à titre illustratif et non limitatif.

### Exemple 1 : Etude ultrastructurale des corps lamellaires dans des kératinocytes humains traités par le peptide SEQ ID n°5

Le but de cette étude est d'étudier de manière ultrastructurale, en microscopie électronique à transmission, des kératinocytes traités par le peptide SEQ ID n°5 à 0,5 ppm.

### Protocole :

Des kératinocytes humains normaux en culture sont traités avec une solution à 1% d'une solution mère à 50 ppm du peptide SEQ ID n°5 pendant 48 heures (le milieu en présence de l'actif est changé tous les 24 heures). Les cellules sont lavées au PBS, puis sont fixées par la fixation hypertonique de Karnosky (4% Paraformaldéhyde, 5% glutaraldéhyde dans un tampon 0,08M Phosphate) 1 heure à température ambiante puis 24 heures à 4°C. Les cellules sont détachées du support par grattage, centrifugées 5 minutes à 1000 rpm. Le surnageant est éliminé et un tampon de 1 M sodium cacodylate est déposé sur le culot. Les cellules sont mélangées à 2 % d'agar puis post-fixées par le tétroxyde d'osmium 1 heure. Les spécimens sont alors déshydratés par passages successifs dans une série d'alcool (de 50 à 100 %). Les cellules sont ensuite enrobées dans une résine. La polymérisation s'effectue pendant environ 12 heures à 60°C. Des coupes semi fines de 0,5 µm sont réalisées à l'ultra-microtome. Les coupes sont déposées sur une lame collée à la chaleur puis colorée au bleu de toluidine. Les lames sont ensuite déshydratées de nouveau et montées dans un milieu adéquat. Après avoir choisi la zone d'étude optimale, le bloc est retaillé à la taille désirée et des coupes ultrafines sont alors réalisées, seules les coupes qui ont une couleur « gris-argent » et une taille adéquate sont montées sur la grille de microscopie électronique doublement marquée par de l'uranyl acétate et du citrate de plomb, et sont examinées au microscope à transmission à 60 ou 80 KV.

Résultats : L'étude ultrastructurale montre que l'appareil de Golgi est sensiblement plus développé que dans les cellules témoins. Cette augmentation est liée à une surproduction des corps lamellaires (ou corps d'Odland) qui est le signe d'une augmentation de la synthèse lipidique.

Conclusions : Le peptide SEQ ID n° 5 à 0,5 ppm est capable d'induire une augmentation de la synthèse lipidique dans les kératinocytes humains normaux.

### Exemple 2 : Etude ultrastructurale des « caveolae » dans des fibroblastes humains traités par le peptide SEQ ID n°4

Le but de cette étude est d'étudier au niveau ultrastructural les cavéoles dans les fibroblastes dermiques humains.

Protocole : Des fibroblastes dermiques humains normaux en culture sont traités avec une solution à 1 % d'une solution mère à 50 ppm du peptide SEQ ID n°4 pendant 48 heures (le milieu contenant l'actif est changé tous les 24 heures).

Résultats : l'étude ultrastructurale montre une augmentation importante des cavéoles dans les cellules traitées par le peptide SEQ ID n°4, en comparaison des cellules témoins non traitées. Ces résultats sont le signe d'un effet positif du principe actif, puisque les cavéoles sont des invaginations de la membrane plasmique qui permettent l'externalisation de molécules telles que le cholestérol.

Conclusions: Le peptide SEQ ID n°4 à 0,5 ppm provoque l'augmentation des structures membranaires d'externalisation du cholestérol.

### Exemple 3 : Etude de la différenciation des kératinocytes humains traités par le peptide SEO ID n°5

Le but de cette étude est de déterminer l'influence du peptide SEQ ID n°5 sur la différenciation épidermique.

Protocole : Des kératinocytes humains normaux en culture sont traités avec une solution à 1 % d'une solution mère à 50 ppm du peptide SEQ ID n° 4 pendant 48 heures (le milieu en présence de l'actif est changé tous les 24 heures). Les cellules sont ensuite lavées, fixées au méthanol froid pendant 4 minutes à 4°C. Les cellules sont incubées en présence d'un anticorps anti cyto-pankératines monoclonal au 1/200eme pendant 1 heure à température ambiante puis révélées par un second anticorps au 1/50eme pendant 1 heure à température ambiante, couplées à un marqueur fluorochrome « alexa 488 ». Après montage dans un milieu *ad hoc*, les lames sont observées au microscope à épifluorescence.

Résultats : L'actif augmente l'expression des pankératines dans les cellules traitées.

Conclusions : Le peptide SEQ ID n°5, à 0,5 ppm augmente l'expression des pankératines dans les kératinocytes humains normaux. En présence de peptide SEQ ID n°5, les cellules sont stimulées et en voie de différenciation.

### Exemple 4 : Etude de l'effet protecteur du peptide SEQ ID n°5 sur les cellules cutanées soumises à des rayonnements ultraviolet (UVB)

Le but de cette étude est de déterminer l'effet protecteur du peptide SEQ ID n°5 vis-à-vis de kératinocytes humains normaux soumis à un stress par des rayonnements UVB. Pour cela, des tests de viabilité cellulaire ont été réalisés par la technique au MTT.

Protocole : Les kératinocytes humains normaux sont traités avec une solution à 1 %, d'une solution à 50 ppm de peptide SEQ ID N°5, pendant 24 heures, irradiés par des UVB (50 mJ/cm²) puis cultivés encore 24 heures en présence de la même concentration de peptide SEQ ID n°5. Des contrôles non traités et irradiés sont réalisés dans les mêmes conditions. A la fin de l'expérimentation, les cellules sont incubées dans une solution contenant 0,1 mg/ml de MTT (3-[4, 5-diméthylthiazol-2-yl]-2, 5-diphényltétrazolium, bromure). Ce composé est absorbé par les cellules vivantes puis métabolisé par les enzymes mitochondriales en un composé bleu violet, le formazan, qui sera dosé par spectrophotométrie à 540 nm. La densité optique (D.O.) est alors directement proportionnelle à l'activité enzymatique mitochondriale ainsi qu'au nombre de cellules vivantes.

Résultats: L'évaluation de la viabilité cellulaire par la technique du MTT montre que le peptide SEQ ID n°5, augmente la viabilité cellulaire après irradiation par les UVB de 16 %.

Conclusions: Le peptide SEQ ID n°5, à la concentration de 0,5 ppm, augmente la viabilité cellulaire et protège efficacement les cellules cutanées contre les effets cytotoxiques des rayonnements UVB.

### Exemple 5 : Etude de l'expression de la HMG-CoA réductase dans des biopsies de peau, en présence du peptide SEQ ID n°5

Le but de cette étude est de déterminer l'influence du peptide SEQ ID n°5 sur l'expression de la HMG-CoA réductase.

Protocole : Des échantillons de peau humaine sont mis en culture à l'interface air/liquide. Une solution à 1 %, d'une solution mère à 50 ppm de peptide SEQ ID n°5 est appliquée topiquement, puis les échantillons sont incubés durant 24 heures ou 48 heures.

Ces échantillons de peau sont ensuite fixés avec du formaldéhyde puis inclus dans la paraffine. Des coupes de 2 à 3 µm sont alors réalisées. L'immunomarquage est effectué après démasquage des sites spécifiques par traitement micro-onde puis incubation dans de la trypsine. L'immunomarquage est réalisé à l'aide d'un anticorps polyclonal de lapin spécifique de la HMG-CoA réductase (Millipore, Upstate), puis d'un anticorps secondaire, couplé à un marqueur fluorescent. Les coupes de peau sont alors examinées au microscope à Epi-fluorescence (Nikon Eclipse E600 microscope).

Résultats : Les observations microscopiques montrent une fluorescence plus forte dans les peaux traitées par le peptide SEQ ID n°5, dans les couches supérieures de l'épiderme, par rapport au contrôle non traité.

Conclusions : Le peptide SEQ ID n°5, à la concentration de 0,5 ppm, stimule l'expression de la HMG-CoA réductase, dans les couches supérieures de l'épiderme.

### Exemple 6 : Etude de l'expression de la HMG-CoA réductase dans kératinocytes humains normaux, en présence du peptide SEQ ID n°4

Le but de cette étude est de déterminer l'influence du peptide SEQ ID n°4 sur l'expression de la HMG-CoA réductase dans les kératinocytes humains normaux.

Protocole : Des kératinocytes humains normaux en culture sont traités avec une solution à 1% d'une solution mère à 50 ppm du peptide SEQ ID n°4 pendant 24 ou 48 heures (le milieu contenant l'actif est changé toutes les 24 heures). Les cellules sont ensuite lavées, fixées au méthanol froid pendant 4 minutes à 4°C. Les cellules sont incubées en présence d'un anticorps polyclonal de lapin spécifique de la HMG-CoA réductase (Millipore, Upstate), puis d'un anticorps secondaire, couplé à un marqueur fluorescent. Les cellules sont alors examinées au microscope à Epi-fluorescence (Nikon Eclipse E600 microscope).

Résultats : Les observations microscopiques montrent une fluorescence cytoplasmique plus intense dans les cellules traitées par le peptide SEQ ID n°4.

Conclusions : Le peptide SEQ ID n°4, à la concentration de 0,5 ppm, stimule l'expression de la HMG-CoA réductase, dans les kératinocytes humains normaux.

### Exemple 7 : Préparation de compositions

### 1 - Crème protection solaire:

| Noms commerciaux | Noms INCI | % massique |
|---|---|---|
| PHASE A | | |
| Eau déminéralisée | Aqua (Water) | qsp |
| Pemulen TRI | Acrylates/C10-30 Alkyl Acrylate Crosspolymer | 0,40 |
| Glycerine | Glycerin | 3,00 |
| Nipastat Sodium | Sodium Methylparaben (and) Sodium Ethylparaben (and) Sodium Butyl paraben (and) Sodium Propylparaben (and) Sodium Isobutylparaben | 0,15 |

| PHASE B | | |
|---|---|---|
| Parsol MCX | Ethylhexyl Methoxycinnamate | 7,50 |
| Eusolex 4360 | Benzophenone-3 | 3,00 |
| Parsol 1789 | Butyl Methoxydibenzoylmethane | 2,00 |
| Myritol 318 | Caprylic/Capric Triglyceride | 4,00 |
| Emulgade SEV | Hydrogenated Palm Glycerides (and) Ceteareth-20 (and) Ceteareth-12 (and) Cetearyl Alcohol | 5,00 |
| Propylparaben | Propylparaben | 0,15 |
| Nacol 16-98 | Cetyl Alcohol | 1,00 |

| PHASE C | | |
|---|---|---|
| TEA | Triethanolamine | 0,20 |

| PHASE D | | |
|---|---|---|
| Peptide SEQ ID n° 4 | | 3 ppm |
| Parfum | Parfum (Fragrance) | qsp |
| Colorant | | qsp |

Les constituants de la phase A et de la phase B sont chauffés séparément entre 70°C et 75°C. La phase B est émulsionnée dans la phase A sous agitation. La phase C est ajoutée, à 45°C, en augmentant l'agitation. La phase D est ensuite additionnée lorsque la température se situe en dessous de 40°C. Le refroidissement est poursuivi jusqu'à 25°C sous vive agitation.

### 2-Crème anti-âge :

| ***Noms commerciaux*** | ***Noms INCI*** | % ***massique*** |
|---|---|---|
| ***Phase A*** | | |
| Montanov 68 | Cetearyl Alcohol (and) Cetearyl Glucoside | 6,00 |
| Squalane | Squalane | 3,00 |
| Cetiol SB 45 | Butyrospermum Parkii (Shea Butter) | 2,00 |
| Waglinol 250 | Cetearyl Ethylhexanoate | 3,00 |
| Amerchol L- 101 | Mineral Oil (and) Lanolin Alcohol | 2,00 |
| Abil 350 | Dimethicone | 1,50 |
| BHT | BHT | 0,01 |
| Coenzyme Q10 | Ubiquinone | 0,10 |

| ***Phase B*** | | |
|---|---|---|
| Huile d'Avocat | Persea Gratissima (Avocado) Oil | 1,25 |
| Phenonip | Phenoxyethanol (and) Methylparaben (and) Ethylparaben (and) Butylparaben (and) Propylparaben (and) Isobutylparaben | 0,75 |

| ***Phase C*** | | |
|---|---|---|
| Eau déminéralisée | Aqua (Water) | qsp |
| Butylene Glycol | Butylene Glycol | 2,00 |
| Glucam E10 | Methyl Gluceth-10 | 1,00 |
| Allantoin | Allantoin | 0,15 |
| Carbopol Ultrez 10 | Carbomer | 0,20 |

| ***Phase D*** | | |
|---|---|---|
| TEA | Triethanolamine | 0,18 |

| ***Phase E*** | | |
|---|---|---|
| Peptide SEQ ID n°4 | | 0,5 ppm |
| GP4G | Water (and) Artemia Extract | 1,50 |
| Collaxyl | Water (and) Butylene Glycol (and) Hexapeptide-9 | 3,00 |

| ***Phase F*** | | |
|---|---|---|
| Parfum | Parfum (Fragrance) | qsp |
| Colorant | | qsp |

Préparer et fondre la phase A à 65-70°C. Chauffer la phase C à 65-70°C. La phase B est ajoutée à la phase A juste avant d'émulsionner A dans B. A environ 45°C, le carbomer est neutralisé par addition de la phase D. La phase E est ensuite additionnée sous légère agitation et le refroidissement est poursuivi jusqu'à 25°C. La phase F est alors addi-tionnée si souhaité.

### 3 -Crème protectrice de jour :

| ***Noms commerciaux*** | ***Noms INCI*** | **% *massique*** |
|---|---|---|
| ***Phase A*** | | |
| Emulium Delta | Cetyl alcohol (and) Glyceryl Stearate (and) PEG-75 Stearate (and) Ceteth-20 (and) Steareth-20 | 4,00 |
| Lanette O | Cetearyl Alcohol | 1,50 |
| D C 200 Fluid/100cs | Dimethicone | 1,00 |
| DUB 810C | Coco Caprylate/Caprate | 1,00 |
| DPPG | Propylene Glycol Dipelargonate | 3,00 |
| DUB DPHCC | Dipentaerythrityl Hexacaprylate/Hexacaprate | 1,50 |
| Cegesoft PS6 | Vegetable Oil | 1,00 |
| Vitamine E | Tocopherol | 0,30 |
| Phenonip | Phenoxyethanol (and) Methylparaben (and) Ethylparaben (and) Butylparaben (and) Propylparaben (and) Isobutylparaben | 0,70 |

| ***Phase B*** | | |
|---|---|---|
| Eau déminéralisée | Aqua | qsp 100 |
| Glycerine | Glycerin | 2,00 |
| Carbopol EDT 2020 | Acrylates/C 10-30Alkyl Acrylate Crosspolymer | 0,15 |
| Keltrol BT | Xanthan Gum | 0,30 |

| ***Phase C*** | | |
|---|---|---|
| Sodium Hydroxide (sol.à 10%) | Sodium Hydroxide | 0,30 |

| ***Phase D*** | | |
|---|---|---|
| Eau déminéralisée | Aqua | 5,00 |
| Stay-C 50 | Sodium Ascorbyl Phosphate | 0,50 |

| ***Phase E*** | | |
|---|---|---|
| Butylene Glycol | Butylene Glycol | 2,00 |
| Dekaben CP | Chlorphenesin | 0,20 |

| ***Phase F*** | | |
|---|---|---|
| GP4G | Water (and) Artemia Extract | 1,00 |
| Peptide SEQ ID n°5 | | 5 ppm |

Préparer la phase A et chauffer à 75°C sous agitation. Préparer la phase B en dispersant le carbopol, puis la gomme xanthane sous agitation. Laisser reposer. Chauffer à 75°C.

A température, émulsionner A dans B sous agitation rotor-stator. Neutraliser avec la phase C sous agitation rapide. Après refroidissement à 40°C, additionner la phase D, puis la phase E.

Le refroidissement est poursuivi sous agitation légère et la phase F rajoutée.

## Revendications

1. Peptide dérivé de la HMG-CoA réductase humaine, de formule générale (I)
R₁-(AA)n-X₁-Gly-Glu-Leu-Ser-X₂-X₃-(AA)ₚ-R₂
dans laquelle,
X₁ est l'alanine ou la valine ou l'isoleucine,
X₂ est la leucine ou l'isoleucine ou aucun acide aminé,
X₃ est la méthionine ou la serine ou l'alanine ou aucun acide aminé,
AA représente un acide aminé quelconque, ou un de ses dérivés, et n et p sont des nombres entiers compris entre 0 et 4,
R₁ représente la fonction aminé primaire de l'acide aminé N-terminal, libre ou substituée par un groupement protecteur pouvant être choisi parmi un groupement acétyle, un groupement benzoyle, un groupement tosyle ou un groupement benzyloxycarbonyle,
R₂ représente le groupe hydroxyle de la fonction carboxyle de l'acide aminé C-terminal, libre ou substitué par un groupement protecteur pouvant être choisi parmi une chaîne alkyle de C₁ à C₂₀, ou un groupement NH2, NHY ou NYY avec Y représentant une chaîne alkyle de C₁ à C₄., **caractérisé en ce qu'**il correspond à une des séquences suivantes:
(SEQ ID n°1) Met-Ala-Gly-Glu-Leu-Ser-Leu-Met-Ala-Ala
(SEQ ID n°2) Gly-Val-Gly-Glu-Leu-Ser-Ile-Ser-Ala
(SEQ ID n°3) Ile-Gly-Glu-Leu-Ser-Leu-Ala-Ala
(SEQ ID n°4) Ala-Gly-Glu-Leu-Ser
(SEQ ID n°5) Ala-Gly-Glu-Leu-Ser-NH2
(SEQ ID n°6) Ile-Gly-Glu-Leu-Ser
(SEQ ID n°7) Ile-Gly-Glu-Leu-Ser-NH2.

2. Peptide selon la revendication 1 **caractérisé en ce qu'**il correspond à la séquence SEQ ID n°4.

3. Peptide selon la revendication 1 **caractérisé en ce qu'**il correspond à la séquence SEQ ID n°5.

4. Peptide selon l'une des revendications précédentes **caractérisé en ce qu'**il est solubilisé dans un ou plusieurs solvants physiologiquement acceptables, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

5. Peptide selon l'une des revendications précédentes, **caractérisé en ce qu'**il est utilisé à titre de médicament.

6. Composition cosmétique ou pharmaceutique **caractérisée en ce qu'**elle comprend, dans un milieu physiologiquement acceptable, au moins un peptide tel que défini dans l'une quelconque des revendications 1 à 4, en tant que principe actif, utilisé seul ou en association avec au moins un autre principe actif.

7. Composition cosmétique ou pharmaceutique selon la revendication 6, **caractérisée en ce que** ledit peptide est présent à une concentration comprise entre 0,0005 et 500 ppm.

8. Composition cosmétique ou pharmaceutique selon la revendication 7, **caractérisée en ce que** ledit peptide est présent à une concentration comprise entre 0,01 et 5 ppm.

9. Composition cosmétique ou pharmaceutique selon l'une des revendications 6 à 8, **caractérisée en ce qu'**elle est destinée à une administration par voie topique.

10. Utilisation cosmétique d'une composition comprenant une quantité efficace d'au moins un peptide tel que défini dans l'une quelconque des revendications 1 à 4, en tant que principe actif activateur de la HMG-CoA réductase humaine.

11. Utilisation cosmétique selon la revendication 10 pour renforcer la fonction barrière de l'épiderme et stimuler la différenciation épidermique.

12. Utilisation cosmétique selon la revendication 10 pour prévenir et lutter contre les signes cutanés du vieillissement et du photo-vieillissement.

13. Utilisation cosmétique selon la revendication 10 pour protéger la peau des agressions extérieures.

## Patentansprüche

1. Peptid, das von humaner HMG-CoA-Reduktase abgeleitet ist, mit der allgemeinen Formel (I):
R₁- (AA) n-X₁-Gly-Glu-Leu-Ser-X₂-X₃-(AA)ₚ-R₂,
in der
X₁ Alanin oder Valin oder Isoleucin ist,
X₂ Lysin oder Isoleucin oder keine Aminosäure ist,
X₃ Methionin oder Serin oder Alanin oder keine Aminosäure ist,
AA für eine beliebige Aminosäure oder eines ihrer Derivate steht und n und p ganze Zahlen zwischen 0 und 4 sind,
R₁ für die primäre Aminofunktion der N-terminalen Aminosäure steht, die frei oder durch eine Schutzgruppe substituiert ist, die aus einer Acetylgruppe, einer Benzoylgruppe, einer Tosylgruppe oder einer Benzyloxycarbonylgruppe ausgewählt sein kann,
R₂ für die Hydroxylgruppe der Carboxylfunktion der C-terminalen Aminosäure steht, die frei oder durch eine Schutzgruppe substituiert ist, die aus einer C₁- bis C₂₀-Alkylkette oder einer NH₂-, NHY- oder NYY-Gruppe, wobei Y für eine C₁- bis C₄-Alkylkette steht, ausgewählt sein kann, **dadurch gekennzeichnet, dass** das Peptid einer der folgenden Sequenzen entspricht:
(SEQ ID Nr. 1) Met-Ala-Gly-Glu-Leu-Ser-Leu-Met-Ala-Ala
(SEQ ID Nr. 2) Gly-Val-Gly-Glu-Leu-Ser-Ile-Ser-Ala
(SEQ ID Nr. 3) Ile-Gly-Glu-Leu-Ser-Leu-Ala-Ala
(SEQ ID Nr. 4) Ala-Gly-Glu-Leu-Ser
(SEQ ID Nr. 5) Ala-Gly-Glu-Leu-Ser-NH₂
(SEQ ID Nr. 6) Ile-Gly-Glu-Leu-Ser
(SEQ ID Nr. 7) Ile-Gly-Glu-Leu-Ser-NH₂.

2. Peptid nach Anspruch 1, **dadurch gekennzeichnet, dass** es der Sequenz SEQ ID Nr. 4 entspricht.

3. Peptid nach Anspruch 1, **dadurch gekennzeichnet, dass** es der Sequenz SEQ ID Nr. 5 entspricht.

4. Peptid nach einem der vorhergehenden Ansprüche, **dadurch gekennzeichnet, dass** es in einem oder mehreren physiologisch akzeptablen Lösungsmitteln solubilisiert wird, wie Wasser, Glycerin, Ethanol, Propylenglykol, Butylenglykol, Dipropylenglykol, ethoxylierte oder propoxylierte Diglykole, cyclische Polyole, Vaseline, ein pflanzliches Öl oder jegliches Gemisch dieser Lösungsmittel.

5. Peptid nach einem der vorhergehenden Ansprüche, **dadurch gekennzeichnet, dass** es als Arzneimittel verwendet wird.

6. Kosmetische oder pharmazeutische Zusammensetzung, **dadurch gekennzeichnet, dass** sie in einem physiologisch akzeptablen Medium mindestens ein wie in einem der Ansprüche 1 bis 4 definiertes Peptid als Wirkstoff umfasst, der allein oder in Verbindung mit mindestens einem anderen Wirkstoff verwendet wird.

7. Kosmetische oder pharmazeutische Zusammensetzung nach Anspruch 6, **dadurch gekennzeichnet, dass** das besagte Peptid in einer Konzentration zwischen 0,0005 und 500 ppm vorliegt.

8. Kosmetische oder pharmazeutische Zusammensetzung nach Anspruch 7, **dadurch gekennzeichnet, dass** das besagte Peptid in einer Konzentration zwischen 0,01 und 5 ppm vorliegt.

9. Kosmetische oder pharmazeutische Zusammensetzung nach einem der Ansprüche 6 bis 8, **dadurch gekennzeichnet, dass** sie für eine topische Verabreichung vorgesehen ist.

10. Kosmetische Verwendung einer Zusammensetzung, die eine wirksame Menge eines wie in einem der Ansprüche 1 bis 4 definierten Peptids als Wirkstoff, der humane HMG-CoA-Reduktase aktiviert, umfasst.

11. Kosmetische Verwendung nach Anspruch 10 zum Verstärken der Barrierefunktion der Epidermis und zum Stimulieren der epidermalen Differenzierung.

12. Kosmetische Verwendung nach Anspruch 10 zum Verhindern und Bekämpfen von Zeichen der Alterung und der Lichtalterung der Haut.

13. Kosmetische Verwendung nach Anspruch 10 zum Schützen der Haut vor äußeren Einflüssen.

## Claims

1. Peptide derived from human HMG-CoA reductase, having the general formula (I)
R₁- (AA) n-X₁-Gly-Glu-Leu-Ser-X₂-X₃-(AA)ₚ-R₂
wherein,
X₁ is alanine or valine or isoleucine,
X₂ is leucine or isoleucine or no amino acid,
X₃ is methionine or serine or alanine or no amino acid,
AA represents any amino acid, or one of the derivatives thereof, and n and p are integers between 0 and 4,
R₁ represents the primary amine function of the N-terminal amino acid, free or substituted by a protecting group suitable for being chosen from an acetyl group, a benzoyl group, a tosyl group or a benzyloxycarbonyl group,
R₂ represents a hydroxyl group of the carboxyl function of the C-terminal amino acid, free or substituted by a protecting group suitable for being chosen from a C₁ to C₂₀ alkyl chain, or an NH2, NHY or NYY group where Y represents a C₁ to C₄ alkyl chain, **characterised in that** it corresponds to one of the following sequences:
(SEQ ID No. 1) Met-Ala-Gly-Glu-Leu-Ser-Leu-Met-Ala-Ala
(SEQ ID No. 2) Gly-Val-Gly-Glu-Leu-Ser-Ile-Ser-Ala
(SEQ ID No. 3) Ile-Gly-Gly-Leu-Ser-Leu-Ala-Ala
(SEQ ID No. 4) Ala-Gly-Glu-Leu-Ser
(SEQ ID No. 5) Ala-Gly-Glu-Leu-Ser-NH2
(SEQ ID No. 6) Ile-Gly-Glu-Leu-Ser
(SEQ ID No. 7) Ile-Gly-Glu-Leu-Ser-NH2.

2. Peptide according to claim 1 **characterised in that** it corresponds to the sequence SEQ ID No. 4.

3. Peptide according to claim 1 **characterised in that** it corresponds to the sequence SEQ ID No. 5.

4. Peptide according to any of the above claims **characterised in that** it is solubilised in one or a plurality of physiologically acceptable solvents, such as water, glycerol, ethanol, propylene glycol, butylene glycol, dipropylene glycol, ethyoxylated or propoxylated diglycols, cyclic polyols, petrolatum, a plant oil or any mixture of these solvents.

5. Peptide according to any of the above claims, **characterised in that** it is used as a medicinal product.

6. Cosmetic or pharmaceutical composition **characterised in that** it comprises, in a physiologically acceptable medium, at least one peptide as defined in any of claims 1 to 4, as an active substance, used alone or in association with at least one further active substance.

7. Cosmetic or pharmaceutical composition according to claim 6, **characterised in that** said peptide is present at a concentration between 0.0005 and 500 ppm.

8. Cosmetic or pharmaceutical composition according to claim 7, **characterised in that** said peptide is present at a concentration between 0.01 and 5 ppm.

9. Cosmetic or pharmaceutical composition according to any of claims 6 to 8, **characterised in that** it is intended for topical administration.

10. Cosmetic use of a composition comprising an effective quantity of at least one peptide as defined in any of claims 1 to 4, as an active substance activating human HMG-CoA reductase.

11. Cosmetic use according to claim 10 for strengthening the barrier function of the epidermis and stimulating epidermal differentiation.

12. Cosmetic use according to claim 10 for preventing and combating skin signs of ageing and photo-ageing.

13. Cosmetic use according to claim 10 for protecting the skin from external attacks.
